# EUROPÄISCHE PATENTSCHRIFT

(11) **EP 3 576 800 B1**
(45) Veröffentlichungstag und Bekanntmachung des Hinweises auf die Patenterteilung: **10.03.2021**
(21) Anmeldenummer: 18702219.9
(22) Anmeldetag: 26.01.2018
(51) Int. Cl.: A61L 27/16, A61L 27/44, A61L 27/46, A61L 27/56, A61L 27/58

(54) **KNOCHENERSATZMATERIALKÖRPER UND VERFAHREN ZU DESSEN HERSTELLUNG**
BONE REPLACEMENT MATERIAL BODY AND METHOD FOR PRODUCING THE SAME
CORPS DE MATÉRIAU DE PROTHÈSE OSSEUSE ET SON PROCÉDÉ DE FABRICATION

(30) Priorität: 31.01.2017 DE 102017101804
(43) Veröffentlichungstag der Anmeldung: 11.12.2019
(73) Patentinhaber: Deutsche Institute für Textil- und Faserforschung Denkendorf, 73770 Denkendorf (DE)
(72) Erfinder: OBERHOFFNER, Sven, 71384 Weinstadt (DE); MALUNAT, Katrin, 73733 Esslingen (DE); DAUNER, Martin, 73730 Esslingen (DE)
(74) Vertreter: Rüger Abel Patentanwälte PartGmbB
(86) Internationale Anmeldenummer: PCT/EP2018/051907
(87) Internationale Veröffentlichungsnummer: WO 2018/141639

(56) Entgegenhaltungen:
- WO-A1-2010/044758
- WO-A1-2014/006519
- WO-A2-2007/084609
- US-B1- 6 235 061
- US-B1- 8 071 007
- R. FILMON ET AL: "Non-connected versus interconnected macroporosity in poly(2-hydroxyethyl methacrylate) polymers. An X-ray microtomographic and histomorphometric study", JOURNAL OF BIOMATERIALS SCIENCE. POLYMER EDITION., Bd. 13, Nr. 10, 1. Januar 2002 (2002-01-01), Seiten 1105-1117, XP055467189, NL ISSN: 0920-5063, DOI: 10.1163/156856202320813828
- TADIC D ET AL: "A novel method to produce hydroxyapatite objects with interconnecting porosity that avoids sintering", BIOMATERI, ELSEVIER SCIENCE PUBLISHERS BV., BARKING, GB, Bd. 25, Nr. 16, 1. Juli 2004 (2004-07-01), Seiten 3335-3340, XP004490265, ISSN: 0142-9612, DOI: 10.1016/J.BIOMATERIALS.2003.10.007

## Beschreibung

Die Erfindung betrifft ein Verfahren zur Herstellung eines Knochenersatzmaterialkörpers aus einem Ersatzmaterial. Solche Ersatzmaterialien zur Behandlung von Knocheneffekten können autologe, allogene oder xenogene Ersatzmaterialien sein. Bei diesen Ersatzmaterialien handelt es sich um biologisches Material. Es werden auch synthetische Materialien als Ersatzmaterial verwendet.

Bei den synthetischen Ersatzmaterialien sind Keramiken, Zemente, Polymere oder Komposite bekannt. Bei keramischen Ersatzmaterialien werden häufig Calciumphosphatverbindungen eingesetzt. Bei der Herstellung durch Sintern kann durch hohe Temperaturen die Stabilität der Keramik erhöht werden. Dies hat jedoch zur Folge, dass die Porosität und die Degradationsgeschwindigkeit abnehmen, was die Osteointegration benachteiligt beeinträchtigen kann. Die Dauer der Degradation ist bei calciumphosphatbasierten Ersatzmaterialien nicht genau einstellbar bzw. vorhersehbar und die Degradationsdauer ist vergleichsweise lang. Die üblicherweise verwendeten Zemente beruhen in der Regel ebenfalls auf Calciumphosphat.

Auch der Einsatz von Polymeren als Knochenersatzmaterial ist bekannt. Als Vorteil von Polymeren wird deren Biokompatibilität, vielfältige Verarbeitungs- und Formgebungsmöglichkeiten, geringe Dichte und die hohe Duktilität angesehen. Außerdem sind funktionelle Gruppen verfügbar. Bei der Degradation von Polymeren wird es als problematisch angesehen, dass Monomere oder Oligomere freigesetzt werden können, die wiederum Entzündungsreaktionen auslösen können. Die Verwendung von Biopolymeren, wie beispielsweise Polysaccharide, Kollagen sowie Kollagenderivate, ist insoweit problematisch, als es sich um ein Naturprodukt handelt, das nicht immer in derselben Qualität und Reinheit verfügbar ist (S. Heinemann, M. Gelinsky, H. Worch, T. Hanke, "Resorbierbare Knochenersatzmaterialien", Der Orthopäde 9, 2011, Seiten 761 bis 773).

WO 2007/084609 A2 beschreibt ein poröses Osteoimplantat, das aus Partikeln eines Knochenersatzmaterial hergestellt werden kann, die mit einem biokompatiblen Polymer gemischt werden. Das Gemisch wird anschließend einer Behandlung mit einem überkritischen Fluid unterzogen, um den porösen Verbundwerkstoff zu erhalten.

WO 2010/044758 A1 offenbart ein bioresorbierbares Knochenersatzmaterial mit einer Mehrzahl von Kanälen. Das Knochenersatzmaterial ist durch Schichten aus Mikrofilamentmaschen gebildet, die gedreht versetzt zueinander angeordnet werden.

US 6,235,061 B1 beschreibt einen Knochenersatzmaterialkörper mit einer dreidimensionalen Polymermatrix aus einem biokompatiblen Polyphosphazen.

Weitere Knochenersatzmaterialien sowie ihre Herstellung sind beispielsweise aus US 8,071,007 B1, WO 2010/044758 A1 oder den Artikeln von R. FILMON ET AL: "Non-connected versus interconnected macroporosity in poly(2-hydroxyethyl methacrylate) polymers. An X-ray microtomographic and histomorphometric study", JOURNAL OF BIOMATERIALS SCIENCE. POLYMER EDITION., Bd. 13, Nr. 10, 1. Januar 2002 (2002-01-01), Seiten 1105-1117, XP055467189, NL, ISSN: 0920-5063, DOI: 10.1163/156856202320813828 und D. Tadic et al.: "A novel method to produce hydroxyapatite objects with interconnecting porosity that avoids sintering", Biomat. Bd. 25, Nr. 16, 01-07-2004, 3335-3340, XP004490265 bekannt.

Es kann daher als Aufgabe der Erfindung angesehen werden, ein Verfahren zur Herstellung eines Knochenersatzmaterialkörper mit gutem mechanischen und biologischen Eigenschaften bereit zu stellen, wobei sich dieser außerdem effizient herstellen lässt.

Diese Aufgabe wird durch ein Verfahren mit den Merkmalen des Patentanspruches 1 gelöst.

Der Knochenersatzmaterialkörper besteht aus einem Ersatzmaterial, das ein synthetisches, resorbierbares Polymer aufweist oder aus wenigstens einem solchen Polymer gebildet ist. Als Polymer kann ein Homopolymer oder ein Copolymer verwendet werden. Das Copolymer kann in unterschiedlichen Varianten vorliegen, beispielsweise in Form eines Block-Copolymers. Das für das Ersatzmaterial verwendete Polymer ist insbesondere ein thermoplastisches Polymer und/oder ein nachträglich vernetzbares Polymer.

Innerhalb des Ersatzmaterials ist ein interkonnektierendes Porensystem gebildet bzw. vorhanden. Durch dieses interkonnektierende Porensystem wird eine osteokonduktive Wirkung erzielt, die für das Ersatzmaterial erforderlich ist. Das Ersatzmaterial bildet einen porösen Körper mit dem interkonnektierenden Porensystem. Mit Hilfe dieses Porensystems wird die Möglichkeit geschaffen, dass biologische Materialien (Kapillaren, pervaskuläres Gewebe und Knochenzellen) in das synthetische Ersatzmaterial einwachsen. Insbesondere wird durch das Porensystem eine ausreichende Vaskularisation gewährleistet.

Unter einem interkonnektierenden Porensystem ist ein Porensystem zu verstehen, bei dem einzelne Poren keine geschlossene Kavität innerhalb des Knochenersatzmaterialkörpers bilden, sondern die Poren miteinander verbunden sind und dadurch die Interkonnektivität schaffen. Die vielen Porensystemabschnitte bzw. Kanalabschnitte des Porensystems haben im Raum eine beliebige Orientierung und sind isotrop orientiert und verteilt im Knochenersatzmaterialkörper angeordnet.

Es ist nicht erforderlich, dass sämtliche im Knochenersatzmaterialkörper vorhandenen Poren oder Kanalabschnitte miteinander verbunden oder zur Außenfläche des Knochenersatzmaterialkörpers offen sind. Auch mehrere getrennte Porensystemteile können vorhanden sein, die jeweils eine oder mehrere Mündungsstellen an der Außenseite des Knochenersatzmaterialkörpers aufweisen. Es können auch geringere Anteile von vollständig geschlossenen Poren bzw. Kavitäten im Knochenersatzmaterialkörper vorhanden sein, wobei deren Volumenanteil möglichst gering sein soll. Der Volumenanteil aller vollständig geschlossener Poren am Gesamtvolumen des Porensystems beträgt insbesondere weniger als 15%, vorzugsweise weniger als 10% und weiter vorzugsweise weniger als 5%.

Es ist vorteilhaft, wenn das Porensystem aus einzelnen miteinander verbundenen Kanalabschnitten gebildet ist. Einzelne Kanalabschnitte sind dann miteinander verbunden, wenn eine Fluidverbindung von einem Kanalabschnitt zum anderen Kanalabschnitt besteht. Die Kanalabschnitte bzw. die Poren des Porensystems haben vorteilhafterweise einen Durchmesser bzw. eine Querschnittsabmessung im Bereich von 150 Mikrometern bis 500 Mikrometern und weiter vorzugsweise im Bereich von 250 Mikrometern bis 400 Mikrometern. Zumindest ist der Teil der Poren bzw. der Kanalabschnitte des Porensystems, deren Querschnittsabmessung sich innerhalb des angegebenen Bereichs befindet, größer als 80%, vorzugsweise größer als 90% und weiter vorzugsweise größer als 95%.

Das Porensystem ist bevorzugt an einer Vielzahl von Stellen zur Außenfläche des Knochenersatzmaterialkörpers offen, um das Einwachsen in das umgebende Körpergewebe zu verbessern. Unter der Außenfläche des Knochenersatzmaterialkörpers ist die gesamte äußere bzw. von außen zugängliche Fläche des Knochenersatzmaterialkörpers zu verstehen, die sich aus mehreren Teilflächen - z.B. obere Fläche, untere Fläche, eine oder mehrere seitliche Flächen - zusammensetzen kann.

Bei einem vorteilhaften Knochenersatzmaterialkörper beträgt die Porosität des Knochenersatzmaterialkörpers mindestens 60% oder mindestens 70%.

Es ist außerdem vorteilhaft, wenn der Knochenersatzmaterialkörper einen Elastizitätsmodul von mindestens 5 MPa, beispielsweise für spongiöse Knochendefekte, und weiter vorzugsweise von mindestens 10 MPa hat. Durch die verbleibende Elastizität kann der Knochenersatzmaterialkörper bei seiner Verwendung elastisch verformt und an die jeweiligen Gegebenheiten beim operativen Eingriff angepasst werden. Durch eine Vernetzung der polymeren Matrix, beispielsweise durch Gammabestrahlung und/oder Bestrahlung mit UV-Licht und/oder eine andere geeignete Strahlung oder Energiezufuhr, kann der Elastizitätsmodul erhöht werden, so dass das Knochenersatzmaterial auch im lasttragenden Bereich eingesetzt werden kann.

Als Polymer für das Ersatzmaterial kann beispielsweise eines oder mehrere der folgenden Polymere verwendet werden: Glykolid, Laktid, Trimethylencarbonat, Caprolacton und/oder p-Dioxanon oder alle daraus hergestellten Copolymere, vorzugsweise statistische Copolymere oder Blockcopolymere.

Es ist bevorzugt, wenn das resorbierbare Polymer eine Degradationsdauer im Bereich von 1,5 bis 12 Monaten und weiter vorzugsweise im Bereich von 3 bis 8 Monaten aufweist. In dieser Zeitspanne kann sich der Knochen im Bereich des Knochendefekts ausreichend gut regenerieren. Eine vollständige Absorption der gesamten Masse des Knochenersatzmaterialkörpers im menschlichen Körper kann beispielsweise um den Faktor 2 bis 3 größer sein als die Degradationsdauer.

Bei einem bevorzugten Knochenersatzmaterialkörper enthält das Ersatzmaterial zusätzlich zu dem wenigstens einen Polymer ein anorganisches Füllmittel. Dabei kann es sich vorzugsweise um ein keramisches Füllmittel handeln, wie etwa ein Calciumphosphat und/oder Hydroxilapatit und/oder Bioglas. Auch ein Materialgemisch kann als Füllmittel verwendet werden. Durch den Einsatz eines solchen Füllmittels kann das Einwachsverhalten der Knochenzellen und die Stabilität des Knochenersatzmaterialkörpers verbessert werden. Das Füllmittel ist vorzugsweise gleichmäßig in der polymeren Matrix des Ersatzmaterials gemischt bzw. verteilt.

Es ist bevorzugt, wenn der Anteil des Füllmittels im Ersatzmaterial mindestens 10 Gew.-% und vorzugsweise mindestens 40 Gew.-% beträgt. Der maximale Anteil des Füllmittels im Ersatzmaterial kann 80 Gew.-% betragen.

Der vorstehend beschriebene Knochenersatzmaterialkörper lässt sich erfindungsgemäß effizient wie folgt herstellen:
Zunächst wird das Ersatzmaterial bereitgestellt, das ein synthetisches, resorbierbares Polymer aufweist und optional ein Füllmittel, wie etwa ein keramisches Füllmittel enthalten kann. Außerdem wird ein Kernmaterial bereitgestellt. Das Kernmaterial hat insbesondere einen höheren Schmelzpunkt als das Polymer. Aus dem Ersatzmaterial und dem Kernmaterial wird ein Bikomponentenfaden hergestellt, beispielsweise durch Koextrusion. Das Kernmaterial kann in Umfangsrichtung um den Bikomponentenfaden durch das Ersatzmaterial vollständig umschlossen sein. Es sind aber auch andere Anordnungsmöglichkeiten des Ersatzmaterials und des Kernmaterials in dem Bikomponentenfaden möglich.

Der wenigstens eine Bikomponentenfaden oder wenigstens ein Fadenabschnitt davon wird in einer Form angeordnet. Beispielsweise können eine Vielzahl von kurzen Fadenabschnitten eines hergestellten Bikomponentenfadens insbesondere isotrop in eine Werkzeugform eingebracht werden. Es ist auch möglich, aus einem oder mehreren langen Bikomponentenfäden ein dreidiemsionales textiles Gebilde - beispielsweise ein Vliesmaterial, ein Gewebe, ein 3D-Gewirk oder ein 3D-Gewebe - herzustellen, das in der Werkzeugform angeordnet wird. Vorteilhaft ist eine möglichst isotrope Anordnung von einzelnen Fadenabschnitten innerhalb der Werkzeugform.

Das in der Werkzeugform angeordnete Fadenmaterial wird zumindest bis auf und vorzugsweise über den Schmelzpunkt des Ersatzmaterials erwärmt, so dass sich das Ersatzmaterial zu einem Formkörper verbindet. Dabei kann auf das Ersatzmaterial in der Werkzeugform Druck ausgeübt werden. Die Erwärmung des Ersatzmaterials in der Werkzeugform bleibt insbesondere unterhalb des Schmelzpunktes des Kernmaterials, so dass das Kernmaterial seine Struktur beibehält.

Nach dem Herstellen des Formkörpers wird in einem weiteren Schritt das Kernmaterial aus dem Formkörper herausgelöst. Durch das Herauslösen des Kernmaterials entsteht das Porensystem innerhalb des Ersatzmaterials.

Es ist vorteilhaft, wenn das Kernmaterial ein in einem Lösungsmittel lösbares Material ist und das Herauslösen unter Verwendung des Lösungsmittels erfolgt. Insbesondere handelt es sich bei dem Kernmaterial um wasserlösliches Material. Das Ersatzmaterial, das zur Abgrenzung gegenüber dem Kernmaterial auch als Mantelmaterial bezeichnet werden kann, ist beständig gegenüber dem Lösungsmittel und wird während des Einwirkens des Lösungsmittels auf das Kernmaterial durch das Lösungsmittel nicht oder nur unwesentlich gelöst. Das Ersatzmaterial bzw. Mantelmaterial ist daher im verwendeten Lösungmittel unlöslich oder schlecht löslich.

Vorzugsweise wird als Kernmaterial Polyvinylalkohol (PVA) verwendet. PVA hat einen hohen Schmelzpunkt von etwa 180°C und ist biokompatibel. Mögliche verbleibende Reste im Knochenersatzmaterial sind daher unkritisch, wenn das Kernmaterial nicht vollständig aus dem Formkörper herausgelöst werden kann.

Das Herauslösen des Kernmaterials kann unter Erwärmung des Formkörpers durchgeführt werden, wobei die Temperatur unterhalb des Schmelzpunktes des Ersatzmaterials gehalten wird, um dessen Form beizubehalten und ein Kollabieren des Porensystems zu verhindern. Beispielsweise kann der Formkörper bei etwa 40 bis 50°C in ein Lösungsmittelbad getaucht werden, um das Kernmaterial herauszulösen.

Nach dem Herauslösen des Kernmaterials unter Verwendung eines Lösungsmittels, kann der hergestellte Knochenersatzmaterialkörper in einem weiteren Verfahrensschritt getrocknet und/oder aufgereinigt werden.

Zur Erhöhung der mechanischen Festigkeit bzw. des Elastizitätsmoduls des hergestellten Knochenersatzmaterialkörpers kann in einem weiteren Verfahrensschritt bei einem bevorzugten Verfahren eine physikalische und/oder chemische Vernetzung des Polymers erfolgen, beispielsweise durch eine Strahlungseinwirkung mit Beta- und/oder Gammastrahlung und/oder durch Aufbringen eines chemischen Vernetzungsmittels. Beispielsweise kann das Ersatzmaterial bzw. Mantelmaterial vor oder im Rahmen der Bikomponentenfaserherstellung mit einem UV-initiierbaren Vernetzen additiviert werden.

Vorteilhafte Ausgestaltungen des Verfahrens ergeben sich aus den abhängigen Patentansprüchen, der Beschreibung und der Zeichnungen. Nachfolgend werden bevorzugte Ausführungsbeispiele der Erfindung anhand der beigefügten Zeichnungen im Einzelnen erläutert. Es zeigen:
Figur 1 eine schematische Darstellung eines als Ersatzmaterial für den Knochenersatzmaterialkörper verwendeten Block-Copolymers,
Figur 2 eine stark schematisierte blockschaltbildähnliche Darstellung einer Vorrichtung zur Herstellung eines Bikomponentenfadens,
Figuren 3a bis 3f unterschiedliche Querschnittsausgestaltungen an einer Austrittsöffnung der Vorrichtung aus Figur 2,
Figur 4 eine schematische perspektivische Darstellung eines hergestellten Bikomponentenfadens,
Figur 5 eine Mehrzahl von einzelnen Fadenabschnitten des Bikomponentenfadens aus Figur 4,
Figur 6 eine stark schematisierte Prinzipdarstellung eines Formwerkzeugs zur Herstellung eines Formkörpers,
Figur 7 eine schematische Darstellung des Herauslösens des Kernmaterials aus einem hergestellten Formkörper,
Figur 8 ein Ausführungsbeispiel eines hergestellten Knochenersatzmaterialkörpers,
Figur 9 eine CT-Aufnahme eines Abschnitts des Porensystems eines hergestellten Knochenersatzmaterialkörpers,
Figur 10 eine CT-Aufnahme eines Abschnitts eines Porensystems eines weiteren Ausführungsbeispiels eines hergestellten Knochenersatzmaterialkörpers und
Figur 11 ein Flussdiagramm eines bevorzugten Verfahrens zum Herstellen eines Knochenersatzmaterialkörpers.

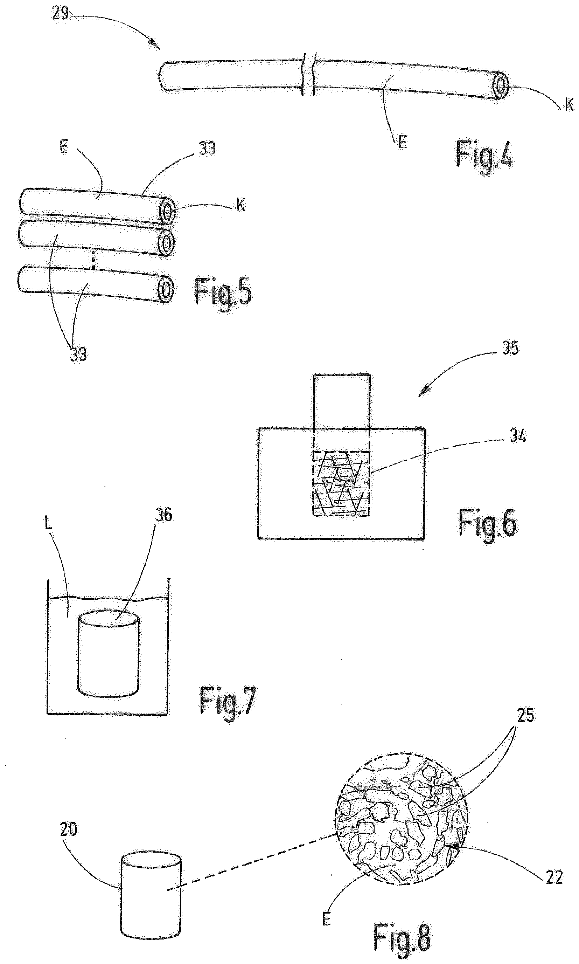

Die Erfindung betrifft ein Verfahren 21 zur Herstellung eines Knochenersatzmaterialkörpers 20. Der Knochenersatzmaterialkörper 20 wird zur Behandlung von Knochendefekten verwendet und kann beispielsweise im Rahmen einer Operation an der Defektstelle des Knochens eingesetzt werden. In Figur 8 ist lediglich beispielhaft und schematisch ein Knochenersatzmaterialkörper 20 mit einer zylindrischen Gestalt veranschaulicht. Die Kontur bzw. Gestalt des Knochenersatzmaterialkörpers 20 kann abhängig vom Anwendungsfall beliebig gewählt werden.

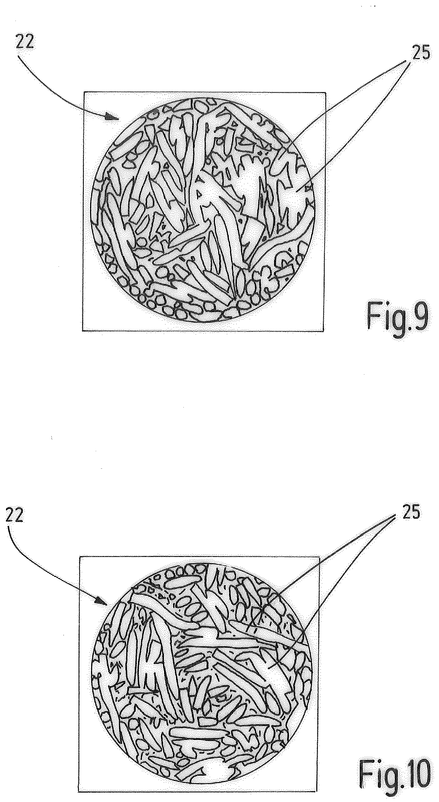

Der Knochensatzmaterialkörper 20 besteht aus einem Skelett aus einem Ersatzmaterial E, das wenigstens ein synthetisches, resorbierbares Polymer P aufweist. In diesem Skelett aus dem Ersatzmaterial E ist ein interkonnektierendes Porensystem 22 vorhanden bzw. gebildet. Das Porensystem 22 ist in der Lichtmikroskopaufnahme in Figur 8 sowie in den Elektronenmikroskopaufnahmen in den Figuren 9 und 10 erkennbar. Das Porensystem 22 ist in den Figuren 9 und 10 durch die dunklen Bereiche innerhalb einer Querschnittsfläche des Skeletts aus dem hell dargestellten Ersatzmaterial E zu erkennen.

Das Ersatzmaterial E kann ein oder mehrere synthetische, resorbierbare Polymeren P aufweisen oder aus einem oder mehreren synthetischen, resorbierbaren Polymeren P bestehen. Als Polymer P kann jedes Homopolymer oder Copolymer, insbesondere Block-Copolymer, verwendet werden, das vorzugsweise aus einem oder mehreren der folgenden Monomere gebildet ist: Glykolid und/oder Laktid und/oder Trimethylencarbonat und/oder Caprolacton und/oder p-Dioxanon.

Als Polymer P können z.B. auch Polyanhydride, 4-Polyhydroxybuttersäure (4-PHB) oder 3-Polyhydroxybuttersäure (3-PHB) verwendet werden.

Das im Ersatzmaterial E verwendete Polymer hat beim Ausführungsbeispiel eine Degradationsdauer von etwa 1,5 bis 12 Monaten und vorzugsweise im Bereich von 3 bis 8 Monaten. Als Degradationsdauer wird die Zeitdauer angesehen, bis zum vollständigen Verlust der mechanischen Festigkeit des Polymers P.

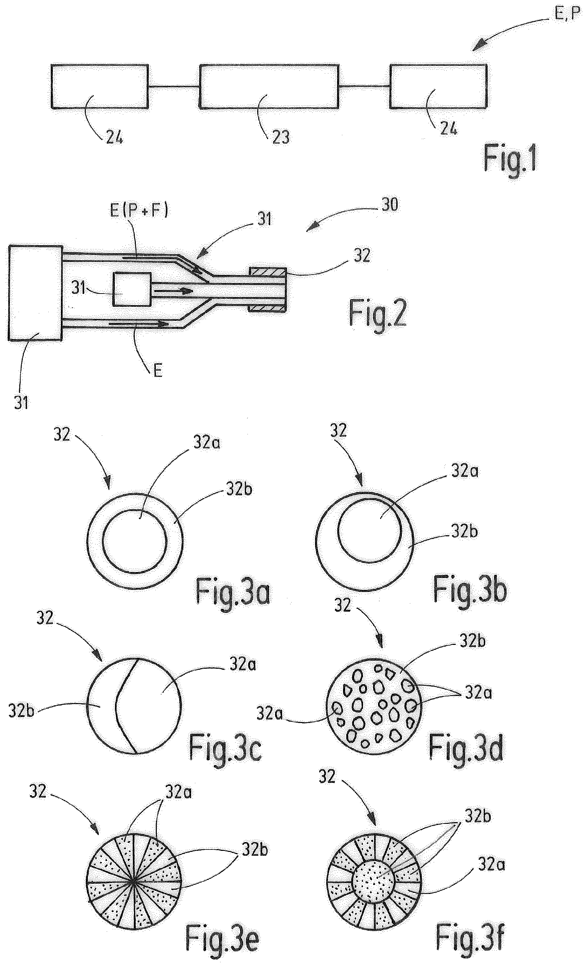

In Figur 1 ist beispielhaft ein bevorzugtes Polymer P in Form eines Block-Copolymers veranschaulicht. Es weist Caprolacton und Trimethlyencarbonat auf. Das amorphe Weichsegment 23 besteht aus Trimethlyencarbonat und Caprolacton, beispielsweise 67,5% Trimethlyencarbonat und 32,5% Caprolacton. Das kristalline Hartsegment des Polymers P besteht aus Caprolacton. Das Polymer P gemäß Figur 1 besteht zu 30% aus dem amorphen Weichsegment und zu 70% aus dem kristallinen Hartsegment.

Der Schmelzpunkt des verwendeten Polymers ist vorzugsweise kleiner als 140°C und beispielsgemäß bei 54°C. Das im Ersatzmaterial E enthaltene wenigstens eine Polymer P hat bevorzugt einen Restmonomergehalt von weniger als 5 Gew.-% und insbesondere weniger als 2 Gew.-%.

Wie erläutert, kann das Ersatzmaterial E ausschließlich aus einem oder mehreren Polymeren P gebildet sein. Bei einem bevorzugten Ausführungsbeispiel enthält das Ersatzmaterial E zusätzlich ein anorganisches Füllmittel F bzw. einen Füllstoff. Das Füllmittel F kann beispielsweise durch wenigstens ein keramisches Material gebildet werden wie etwa Tricalciumphosphat (TCP) oder Hydroxylapatit (HA). Der Anteil des Füllmittels F am Ersatzmaterial E kann im Bereich von 10 Gew.-% bis 80 Gew.-% oder bevorzugt von 40 Gew.-% bis 80 Gew.-% liegen. Ein anorganisches Füllmittel F kann ein verbessertes Einwachsen von Knochenzellen in den Knochenersatzmaterialkörper bewirken. Weiterhin kann das Polymer P zusätzlich Hilfsstoffe zur Vernetzung des Polymers, z.B. UV-Initiatoren enthalten.

Bei dem bevorzugten Ausführungsbeispiel beträgt die Porosität des Knochenersatzmaterialkörpers 20 mindestens 60% oder mindestens 70%. Der Elastizitätsmodul ist bevorzugt größer als 5 MPa, beispielsweise beim Einsatz für spongiöse Knochendefekte, und bei anderen Anwendungen bevorzugt größer als 10 Mpa.

Der Durchmesser bzw. die Querschnittsabmessung der Poren bzw. der Kanalabschnitte 25 des Porensystems 22 rechtwinklig zur jeweiligen Erstreckungsrichtung des Kanalabschnitts 25 betrachtet, beträgt vorzugsweise mindestens 150 Mikrometer und weiter vorzugsweise mindestens 250 Mikrometer. Die Querschnittsabmessung kann beispielsweis bis zu 400 Mikrometern oder bis zu 500 Mikrometern groß sein.

Eine Vielzahl von Kanalabschnitten 25 des Porensystems haben Mündungen an der Außenfläche bzw. Außenseite des hergestellten Knochenersatzmaterialkörpers 20 (schematische Darstellung in Figur 8).

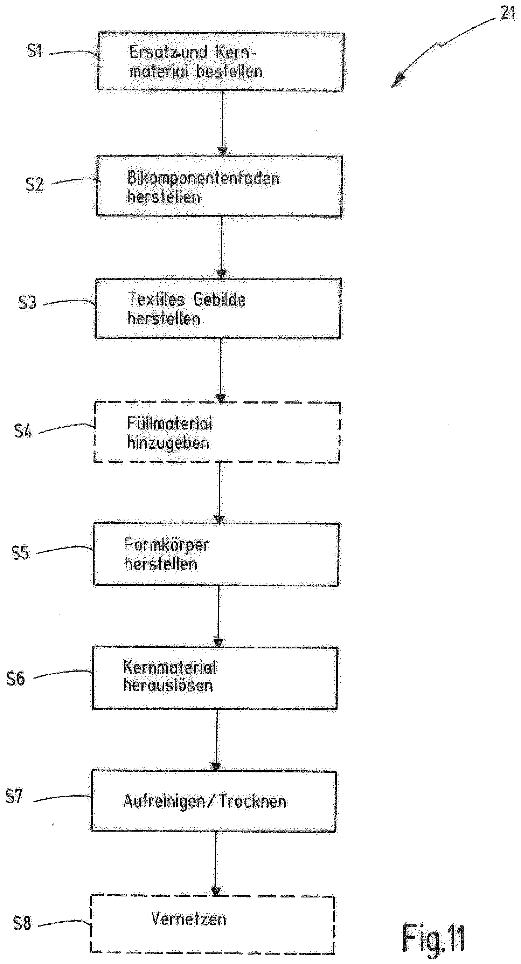

Der Knochenersatzmaterialkörper 20 wird durch das erfindungsgemäße Verfahren 21 gemäß Figur 11 hergestellt.

In einem ersten Verfahrensschritt S1 wird zunächst das Ersatzmaterial E bereitgestellt. Zusätzlich wird ein Kernmaterial K bereitgestellt. Das Kernmaterial K ist vorzugsweise durch ein Lösungsmittel löslich und beispielsgemäß wasserlöslich. Das Kernmaterial K hat einen höheren Schmelzpunkt als das Ersatzmaterial E. Das Ersatzmaterial E ist im Lösungsmittel des Kernmaterials K unlöslich und beispielsgemäß nicht wasserlöslich. Als Kernmaterial K wird beim Ausführungsbeispiel ein Polyvinylalkohol (PVA oder PVOA) verwendet. Bei einem Ausführungsbeispiel wird als Polyvinylalkohol das Polymer "Mowiflex TC232" der Firma Kuraray verwendet. Das Ersatzmaterial E kann wie vorstehend beschrieben gefüllt bzw. additiviert sein.

In einem zweiten Verfahrensschritt S2 wird ein Bikomponentenfaden 29 (Figur 4) hergestellt, der aus dem Ersatzmaterial E und dem Kernmaterial K besteht. Beispielsweise kann das Ersatzmaterial E einen Mantel um das Kernmaterial K bilden.

In Figur 2 ist schematisch eine Vorrichtung 30 zur Koextrusion dargestellt. Einem Extruder 31 werden das Ersatzmaterial E und einem weiteren Extruder 31 das Kernmaterial K zugeführt und die Materialien werden in den Extrudern 31 geschmolzen und aus einer Bikomponentenspinndüse 32 herausgedrückt. Die Bikomponentenspinndüse 32 hat eine vorgegebene Querschnittgestalt, so dass der Bikomponentenfaden 29 den gewünschten Aufbau erhält. Die Bikomponentenspinndüse 32 hat wenigstens einen ersten Öffnungsquerschnitt 32a, aus dem das Kernmaterial K austritt, sowie wenigstens einen zweiten Öffnungsquerschnitt 32b, aus dem das Ersatzmaterial E austritt. Die Anzahl und Anordnung der verschiedenen Öffnungsquerschnitte 32a, 32b kann variieren.

In den Figuren 3a bis 3f sind unterschiedliche Anordnungen und Ausgestaltungen der Bikomponentenspinndüse 32 veranschaulicht. Die Bikomponentenspinndüse 32 gemäß Figur 3a hat einen kreisförmigen zentralen ersten Öffnungsquerschnitt 32a und einen ringförmigen zweiten Öffnungsquerschnitt 32b, der den ersten Öffnungsquerschnitt 32a koaxial umgibt. In Figur 3b ist eine Abwandlung der Ausgestaltung gemäß Figur 3a gezeigt, bei der der erste Öffnungsquerschnitt 32a exzentrisch innerhalb des ringförmigen zweiten Öffnungsquerschnitts 32b angeordnet ist.

In Figur 3c sind die beiden Öffnungsquerschnitte nebeneinander angeordnet. Sie können gleich oder unterschiedlich groß sein, je nachdem, wie die Materialanteile des Ersatzmaterials und des Kernmaterials im Bikomponentenfaden 29 verteilt sein sollen.

Die Ausgestaltung gemäß Figur 3d wird auch als "Inseln im Meer" bezeichnet, wobei eine Mehrzahl von beliebig konturieren ersten Öffnungsquerschnitten 32a innerhalb des die ersten Öffnungsquerschnitte 32a umgebenden zweiten Öffnungsquerschnitt 32b angeordnet sind.

Figur 3e zeigt die Unterteilung der Bikomponentenspinndüse 32 in segmentförmige erste und zweite Öffnungsquerschnitte 32a, 32b. Diese Form kann auch mit einer kreisrunden konzentrischen zusätzlichen Öffnung kombiniert werden (Figur 3f).

Im Prinzip besteht bei der Ausführung der Bikomponentenspinndüse 32 eine große Gestaltungsfreiheit. Zur Herstellung des Bikomponentenfadens aus Figur 4 wurde die Bikomponentenspinndüse 32 gemäß Figur 3a verwendet.

In einem dritten Verfahrensschritt S3 wird aus wenigstens einem hergestellten Bikomponentenfaden 29 oder aus wenigstens einem Fadenabschnitt 33 des Bikomponentenfadens 29 ein textiles Gebilde erzeugt. Beispielsweise können eine oder mehrere Bikomponentenfäden 29 zu einem Vlies, zu einer Gewebelage, zu einem 3D-Gewirk oder einem 3D-Gewebe verschlugen bzw. verbunden werden. Bei dem hier vorgesehenen Ausführungsbeispiel wird der Bikomponentenfaden 29 in eine Vielzahl von relativ kurzen Fadenabschnitten 33 geschnitten. Die Fadenabschnitte 33 können eine Länge von mindestens etwa 2-5 mm aufweisen. Diese kurzen Fadenabschnitte 33 können in eine Werkzeugform 34 eines Formwerkzeugs 35 isotrop orientiert nach Art einer Schüttung angeordnet werden. In diese Werkzeugform 32 kann alternativ das textile Gebilde (Vlies, Gewebe, usw.) angeordnet werden. Vorteilhaft ist, wenn die Orientierung der Fadenabschnitte 33 bzw. des wenigstens einen Bikomponentenfadens 29 innerhalb des textilen Gebildes möglichst isotrop verteilt in verschiedene Raumrichtungen weist, was für die Bildung des interkonnektierenden Porensystems 22 vorteilhaft ist.

Wenn zur Herstellung des Bikomponentenfadens 29 lediglich die polymere(n) Komponente(n) verwendet wurde(n), kann in einem optionalen vierten Verfahrensschritt S4 das Füllmittel F gemeinsam mit der Vielzahl von Fadenabschnitten 33 und/oder dem textilen Gebilde aus wenigstens einem Bikomponentenfaden 29 in die Werkzeugform 34 eingebracht werden. Bei dem hier beschriebenen bevorzugten Ausführungsbeispiel wird das Füllmittel F gemeinsam mit dem Polymer P zur Bildung des Ersatzmaterials E beim Extrudieren des Bikomponentenfadens 29 verwendet, so dass der vierte Verfahrensschritt S4 entfallen kann.

Anschließend wird im nächsten Schritt (fünfter Verfahrensschritt S5) das textile Gebilde bzw. die Mehrzahl von Fadenabschnitten 33 in der Werkzeugform 34 über den Schmelzpunkt des Polymers P erwärmt. Dadurch verbinden sich die einzelnen Fadenabschnitte 33 oder die aneinander anliegenden Teile des textilen Gebildes zu einer Polymermatrix, in der beispielsgemäß das Füllmittel F vorzugsweise gleichmäßig verteilt enthalten ist. In dem Formwerkzeug 35 kann das textile Gebilde bzw. können die Mehrzahl von Fadenabschnitten 33 während des Schmelzens mit Druck beaufschlagt bzw. gepresst werden, was zu einem kompakteren Aufbau des Ersatzmaterials E führt, so dass der hergestellte Knochenersatzmaterialkörper 20 eine höhere Dichte und geringere Porosität aufweist.

Bei diesem Aufschmelzen des Polymers P bleibt die Temperatur unterhalb der Schmelztemperatur des Kernmaterials K.

Der derart hergestellte Formkörper 36 (Figur 7) enthält sowohl das Ersatzmaterial E, als auch das Kernmaterial K. In einem weiteren, sechsten Verfahrensschritt S6 wird das Kernmaterial K aus dem Formkörper 36 entfernt. Beim Ausführungsbeispiel wird der Formkörper 36 in ein Bad mit Lösungsmittel L getaucht (Figur 7). Als Lösungsmittel L dient beispielsgemäß Wasser. Das Lösungsmittelbad kann zur Verbesserung des Herauslösens des Kernmaterials K über die Raumtemperatur erwärmt werden, muss aber unterhalb des Schmelzpunktes des Polymers P des Ersatzmaterials E bleiben, um ein Kollabieren des herzustellenden Porensystems 22 zu verhindern. Durch das Herauslösen des Kernmaterials K aus dem Formkörper 36 bleiben dort die Kanalabschnitte 25, die fluidisch miteinander verbunden sind und auf die Weise das interkonnektierende Porensystem 22 bilden.

Nach dem Herauslösen des Kernmaterials K wird der hergestellt Knochenersatzmaterialkörper 20 in einem siebten Verfahrensschritt S7 z.B. nach einer Aufreinigung getrocknet.

In den Figuren 9 und 10 ist jeweils eine CT-Aufnahme des Porensystems 22 mit einer Vielzahl von Kanalabschnitten 25 in einer Schnittebene durch ein betreffendes Ausführungsbeispiel des Knochenersatzmaterialkörpers 20 veranschaulicht. Bei dem Ausführungsbeispiel gemäß Figur 9 steht das Ersatzmaterial E nur aus einem Polymer P. Beim Ausführungsbeispiel gemäß Figur 10 ist das in der Matrix aus dem Polymer P enthaltene Füllmittel F zu erkennen.

Schließlich kann optional in einem achten Verfahrensschritt S8 zur Erhöhung der mechanischen Festigkeit bzw. des Elastizitätsmoduls des hergestellten Knochenersatzmaterialkörpers 20 eine physikalische und/oder bevorzugt chemische Vernetzung des Polymers P durchgeführt werden. Dies kann beispielsweise durch eine Strahlungseinwirkung mit Beta- und/oder Gammastrahlung und/oder durch Aufbringen eines chemischen Vernetzungsmittels bewirkt werden.

Die Erfindung betrifft ein Verfahren zur Herstellung eines Knochenersatzmaterialkörpers 20. Der Knochenersatzmaterialkörper 20 besteht aus einem Skelett, in dem ein interkonnektierendes Porensystem 22 mit einer Mehrzahl von fluidisch miteinander verbundenen Kanalabschnitten 25 vorhanden bzw. gebildet ist. Das Skelett besteht aus Ersatzmaterial E. Das Ersatzmaterial E weist eine Matrix aus einem synthetischen, resorbierbaren Polymer P auf. Das Ersatzmaterial E kann außerdem ein in der Polymeren Matrix verteilt angeordnetes anorganisches Füllmittel F enthalten. Das Porensystem 22 wird insbesondere dadurch gebildet, dass in einem hergestellten Formkörper 36, der ein Zwischenprodukt darstellt, ein Kernmaterial K in jedem Kanalabschnitt 25 enthalten ist, das aus dem Formkörper 36 herausgelöst wird, insbesondere durch ein Lösungsmittel L.

### Bezugszeichenliste:

- 20: Knochenersatzmaterialkörper
- 21: Verfahren
- 22: Porensystem
- 23: Weichsegment
- 24: Hartsegment
- 25: Kanalabschnitt

- 29: Bikomponentenfaden
- 30: Vorrichtung zum Koextrudieren
- 31: Extruder
- 32: Bikomponentenspinndüse
- 32a: erster Öffnungsquerschnitt
- 32b: zweiter Öffnungsquerschnitt
- 33: Fadenabschnitt
- 34: Form
- 35: Formwerkzeug
- 36: Formkörper

- E: Ersatzmaterial
- K: Kernmaterial
- L: Lösungsmittel
- P: Polymer

- S1: erster Verfahrensschritt
- S2: zweiter Verfahrensschritt
- S3: dritter Verfahrensschritt
- S4: vierter Verfahrensschritt
- S5: fünfter Verfahrensschritt
- S6: sechster Verfahrensschritt
- S7: siebter Verfahrensschritt
- S8: achter Verfahrensschritt

## Patentansprüche

1. Verfahren zur Herstellung eines Knochenersatzmaterialkörpers (20) mit folgenden Schritten:
- Bereitstellen eines Ersatzmaterials (E), das ein synthetisches, resorbierbares Polymer (P) aufweist,
- Bereitstellen eines Kernmaterials (K),
- Herstellen wenigstens eines Bikomponentenfadens (29) aus dem Ersatzmaterial (E) und dem Kernmaterial (K),
- Anordnen eines textilen Gebildes aus wenigstens einem Bikomponentenfaden (29) und/oder einer Vielzahl von Fadenabschnitten (33) eines Bikomponentenfadens (29) in einer Form (34) und Erwärmen des textilen Gebildes zumindest bis auf den Schmelzpunkt des Ersatzmaterials (E), so dass sich das Ersatzmaterial (E) zu einem Formkörper (36) verbindet,
- Herauslösen des Kernmaterials (K) aus dem Formkörper (36) .

2. Verfahren nach Anspruch 1,
**dadurch gekennzeichnet, dass** das Kernmaterial (K) in einem Lösungsmittel (L) lösbar ist und das Herauslösen des Kernmaterials (K) aus dem Formkörper (36) unter Verwendung des Lösungsmittels (L) erfolgt.

3. Verfahren nach Anspruch 1 oder 2,
**dadurch gekennzeichnet, dass** das Kernmaterial (K) einen Volumenanteil im Bereich von 60% bis 85% oder im Bereich von 65% bis 75% am Gesamtvolumen aus Ersatzmaterial (E) und Kernmaterial (K) hat.

4. Verfahren nach einem der vorhergehenden Ansprüche, **dadurch gekennzeichnet, dass** das textile Gebilde eine Schüttung aus Fadenabschnitten (33) des Bikomponentenfadens (29) ist oder dass das textile Gebilde ein zwei- oder dreidimensionales gewebtes, gestricktes oder gewirktes Gebilde oder ein Vliesmaterialgebilde ist, das aus wenigstens einem Bikomponentenfaden (29) hergestellt ist.

5. Verfahren nach einem der vorhergehenden Ansprüche, **dadurch gekennzeichnet, dass** in dem Knochenersatzmaterialkörper (20) ein interkonnektierendes Porensystem (22) gebildet ist.

6. Verfahren nach Anspruch 5,
**dadurch gekennzeichnet, dass** das Porensystem (22) miteinander verbundene Kanalabschnitte (25) aufweist.

7. Verfahren nach Anspruch 5 oder 6,
**dadurch gekennzeichnet, dass** die Kanalabschnitte (25) eine Querschnittsabmessung im Bereich von 150 µm bis 500 µm aufweisen.

8. Verfahren nach einem der Ansprüche 5 bis 7,
**dadurch gekennzeichnet, dass** die Kanalabschnitte (25) eine Querschnittsabmessung im Bereich von 250 µm bis 400 µm aufweisen.

9. Verfahren nach einem der vorhergehenden Ansprüche, **dadurch gekennzeichnet, dass** das Porensystem (22) zu einer Außenfläche des Knochenersatzmaterialkörpers (20) offen ist.

10. Verfahren nach einem der vorhergehenden Ansprüche, **dadurch gekennzeichnet, dass** die Porosität des Knochenersatzmaterialkörpers (20) mindestens 60% oder mindestens 70% beträgt.

11. Verfahren nach einem der vorhergehenden Ansprüche, **dadurch gekennzeichnet, dass** der Elastizitätsmodul des Knochenersatzmaterialkörpers (20) mindestens 5 MPa oder mindestens 10 MPa beträgt.

12. Verfahren nach einem der vorhergehenden Ansprüche, **dadurch gekennzeichnet, dass** das Polymer (P) aus Glykolid und/oder Laktid und/oder Trimethylencarbonat und/oder Caprolacton und/oder p-Dioxanon gebildet ist.

13. Verfahren nach einem der vorhergehenden Ansprüche, **dadurch gekennzeichnet, dass** das Polymer (P) eine Degradationsdauer im Bereich von 1,5 bis 12 Monate aufweist.

14. Verfahren nach einem der vorhergehenden Ansprüche, **dadurch gekennzeichnet, dass** das Polymer (P) eine Degradationsdauer im Bereich von 3 bis 8 Monate aufweist.

15. Verfahren nach einem der vorhergehenden Ansprüche, **dadurch gekennzeichnet, dass** das Ersatzmaterial (E) zusätzlich zu dem Polymer (P) ein anorganischen Füllmittel (F) aufweist.

16. Verfahren nach Anspruch 15,
**dadurch gekennzeichnet, dass** der Anteil des Füllmittels (F) mindestens 10 Gew.-% oder mindestens 40 Gew.-% des Ersatzmaterials beträgt und/oder dass der Anteil des Füllmittels höchstens 80 Gew.-% des Ersatzmaterials (E) beträgt.

17. Verfahren nach Anspruch 15 oder 16,
**dadurch gekennzeichnet, dass** das Füllmittel (F) ein keramisches Material aufweist.

## Claims

1. Method for producing a bone replacement material body (20) with the following steps:
- provision of a replacement material (E) which comprises a synthetic, resorbable polymer (P),
- provision of a core material (K),
- production of at least one bicomponent thread (29) from the replacement material (E) and the core material (K),
- arrangement of a textile fabric of at least one bicomponent thread (29) and/or a multiplicity of thread portions (33) of a bicomponent thread (29) in a mould (34), and heating of the textile fabric at least until reaching a melting point of the replacement material (E) so that the replacement material (E) connects to a mould body (36),
- detaching the core material (K) from the mould body (36).

2. Method according to claim 1, **characterised in that** the core material (K) is soluble in a solvent (L) and the core material (K) is detached from the mould body (36) using the solvent (L).

3. Method according to claim 1 or 2, **characterised in that** the core material (K) has a volume proportion in the range from 60% to 85% or in the range from 65% to 75% of the total volume of the replacement material (E) and core material (K).

4. Method according to any of the preceding claims, **characterised in that** the textile fabric is a bulk pile of fibre portions (33) of the bicomponent thread (29), or the textile fabric is a two- or three-dimensional, woven, knitted or worked fabric or a fleece material fabric which is made from at least one bicomponent thread (29).

5. Method according to any of the preceding claims, **characterised in that** an interconnecting pore system (22) is formed in the bone replacement material body (20).

6. Method according to claim 5, **characterised in that** the pore system (22) comprises interconnected channel portions (25).

7. Method according to claim 5 or 6, **characterised in that** the channel portions (25) have a cross-sectional dimension in the range from 150 µm to 500 µm.

8. Method according to one of claims 5 to 7, **characterised in that** the channel portions (25) have a cross-sectional dimension in the range from 250 µm to 400 µm.

9. Method according to any of the preceding claims, **characterised in that** the pore system (22) is open towards an outer face of the bone replacement material body (20).

10. Method according to any of the preceding claims, **characterised in that** the porosity of the bone replacement material body (20) is at least 60% or at least 70%.

11. Method according to any of the preceding claims, **characterised in that** the modulus of elasticity of the bone replacement material body (20) is at least 5 MPa or at least 10 MPa.

12. Method according to any of the preceding claims, **characterised in that** the polymer (P) is formed from glycolide and/or lactide and/or trimethylene carbonate and/or caprolactone and/or p-dioxanone.

13. Method according to any of the preceding claims, **characterised in that** the polymer (P) has a degradation period in the range from 1.5 to 12 months.

14. Method according to any of the preceding claims, **characterised in that** the polymer (P) has a degradation period in the range from 3 to 8 months.

15. Method according to any of the preceding claims, **characterised in that** the replacement material (E) comprises, in addition to the polymer (P), an inorganic filler (F).

16. Method according to claim 15, **characterised in that** the proportion of filler (F) is at least 10% by weight or at least 40% by weight of the replacement material, and/or the proportion of filler is most 80% by weight of the replacement material (E).

17. Method according to claim 15 or 16, **characterised in that** the filler (F) comprises a ceramic material.

## Revendications

1. Procédé pour la fabrication d'un corps (20) à base de matériau de substitution osseuse, comportant les étapes suivantes :
- fourniture d'un matériau de substitution (E) qui comporte un polymère (P) synthétique, résorbable,
- fourniture d'un matériau d'âme (K),
- production d'au moins un fil bicomposant (29) à base du matériau de substitution (E) et du matériau d'âme (K)
- disposition dans un moule (34) d'une structure textile à partir d'au moins un fil bicomposant (29) et/ou d'une pluralité de segments de fil (33) d'un fil bicomposant (29) et chauffage de la structure textile au moins jusqu'au point de fusion du matériau de substitution (E), de sorte que le matériau de substitution (E) s'assemble en un corps moulé (36),
- élimination du matériau d'âme (K) à partir du corps moulé (36).

2. Procédé selon la revendication 1,
**caractérisé en ce que** le matériau d'âme (K) est soluble dans un solvant (L) et l'élimination du matériau d'âme (K) à partir du corps moulé (36) s'effectue avec utilisation du solvant (L).

3. Procédé selon la revendication 1 ou 2,
**caractérisé en ce que** le matériau d'âme (K) a une proportion en volume dans la plage de 60 % à 85 % ou dans la plage de 65 % à 75 % par rapport au volume total de matériau de substitution (E) et matériau d'âme (K).

4. Procédé selon l'une quelconque des revendications précédentes, **caractérisé en ce que** la structure textile est un amas de segments de fil (33) du fil bicomposant (29) ou **en ce que** la structure textile est une structure bi- ou tridimensionnelle tissée, tricotée ou maillée ou une structure en matériau non-tissé, qui est produite à partir d'au moins un fil bicomposant (29).

5. Procédé selon l'une quelconque des revendications précédentes, **caractérisé en ce qu'**un système de pores (22) interconnecté est formé dans le corps (20) à base de matériau de substitution osseuse.

6. Procédé selon la revendication 5,
**caractérisé en ce que** le système de pores (22) comporte des segments de canaux (25) reliés entre eux.

7. Procédé selon la revendication 5 ou 6,
**caractérisé en ce que** les segments de canaux (25) présentent une dimension en coupe transversale dans la plage de 150 µm à 500 µm.

8. Procédé selon l'une quelconque des revendications 5 à 7, **caractérisé en ce que** les segments de canaux (25) présentent une dimension en coupe transversale dans la plage de 250 µm à 400 µm.

9. Procédé selon l'une quelconque des revendications précédentes, **caractérisé en ce que** le système de pores (22) est ouvert vers une face externe du corps (20) à base de matériau de substitution osseuse.

10. Procédé selon l'une quelconque des revendications précédentes, **caractérisé en ce que** la porosité du corps (20) à base de matériau de substitution osseuse est d'au moins 60 % ou d'au moins 70 %.

11. Procédé selon l'une quelconque des revendications précédentes, **caractérisé en ce que** le modulé d'élasticité du corps (20) à base de matériau de substitution osseuse est d'au moins 5 MPa ou d'au moins 10 MPa.

12. Procédé selon l'une quelconque des revendications précédentes, **caractérisé en ce que** le polymère (P) est constitué à partir de glycolide et/ou lactide et/ou carbonate de triméthylène et/ou caprolactone et/ou p-dioxanone.

13. Procédé selon l'une quelconque des revendications précédentes, **caractérisé en ce que** le polymère (P) présente une durée de dégradation dans la plage de 1,5 à 12 mois.

14. Procédé selon l'une quelconque des revendications précédentes, **caractérisé en ce que** le polymère (P) présente une durée de dégradation dans la plage de 3 à 8 mois.

15. Procédé selon l'une quelconque des revendications précédentes, **caractérisé en ce que** le matériau de substitution (E) comporte en plus du polymère (P) une charge inorganique (F).

16. Procédé selon la revendication 15,
**caractérisé en ce que** la proportion de la charge (F) représente au moins 10 % en poids ou au moins 40 % en poids du matériau de substitution et/ou **en ce que** la proportion de la charge représente au maximum 80 % en poids du matériau de substitution (E).

17. Procédé selon la revendication 15 ou 16,
**caractérisé en ce que** la charge (F) est une matière céramique.
